# EUROPÄISCHE PATENTSCHRIFT

(11) **EP 0 583 442 B1**
(45) Veröffentlichungstag und Bekanntmachung des Hinweises auf die Patenterteilung: **09.10.1996**
(21) Anmeldenummer: 93903175.3
(22) Anmeldetag: 11.02.1993
(51) Int. Cl.: A61B 17/72

(54) **MARKNAGEL**
MEDULLARY NAIL
CLOU MEDULLAIRE

(30) Priorität: 13.02.1992 DE 4204246; 20.02.1992 DE 4205118
(43) Veröffentlichungstag der Anmeldung: 23.02.1994
(73) Patentinhaber: Pennig, Dietmar, Dr. med., D-50935 Köln (DE)
(72) Erfinder: Pennig, Dietmar, Dr. med., D-50935 Köln (DE)
(74) Vertreter: Habbel, Hans-Georg, Dipl.-Ing.
(86) Internationale Anmeldenummer: DE9300135
(87) Internationale Veröffentlichungsnummer: WO9315679

(56) Entgegenhaltungen:
- DE-A- 3 729 840
- DE-A- 3 921 972
- DE-A- 4 012 995

## Beschreibung

Die Erfindung bezieht sich auf einen Marknagel gemäß dem Oberbegriff des Hauptanspruches.

In der gattungsbildenden DE-B-37 29 840 wird ein Markraumverriegelungsnagel beschrieben, bei dem in den metallischen Nagel nichtmetallische Innenkörper einsetzbar sind, die mit den Bohrungen im Nagel koinzidierende Bohrungen aufweisen, deren Durchmesser aber kleiner als der Durchmesser der Bohrungen im Nagel ist.

Während bei Unterarm- und Oberschenkelnägel kein Problem besteht, diese Nägel in den zugeordneten Knochenabschnitten sicher durch quer zur Nagellängsachse verlaufende Befestigungsschrauben zu halten, besteht bei einem Oberarmnagel (DE 88 11 634 U1) die Gefahr, daß durch Aufbohren der erforderlichen Bohrungen für die Befestigungsschrauben Nerven verletzt werden, so daß bisher eine sichere Festlegung eines Oberarmnagels nicht möglich war.

Der Erfindung liegt daher die Aufgabe zugrunde, einen Marknagel zu schaffen, bei dem es möglich ist, auf das Durchbohren der Cortikalis beiderseits des Nagels zu verzichten, d. h. der Nagel nur einseitig durch einen in die Cortikalis und den Nagel eingetriebenen Befestigungspin oder eine entsprechende Schraube festgelegt wird.

Diese der Erfindung zugrundeliegende Aufgabe wird durch die Lehre des Hauptanspruches gelöst.

Durch diese Maßnahme ist es möglich, nunmehr nur einseitig die Cortikalis des Oberarmes zu durchbohren und durch diese Bohrung eine Schraube oder einen Pin einzuführen, der einerseits sicher in der Cortikalis gehalten wird, andererseits aber in den Einsatz innerhalb des Nagels eingreift, ohne daß er auf der anderen Seite aus dem Nagel wieder vorsteht. Hierdurch wird ein Verletzen oder Beschädigen der empfindlichen Nerven auf der anderen Seite mit Sicherheit ausgeschaltet.

Gemäß der Erfindung ist vorgesehen, daß der Einsatz in einer oder mehreren Blindbohrungen angebracht wird oder in zylindrischen Bohrungen, die auf einem großen Teil ihrer Länge zylindrisch sind, sich dann aber konisch verjüngen, so daß beim Einsetzen des Materials in diese Bohrung keine Hohlräume durch Lufteinschlüsse oder ähnliches entstehen können.

Der Einsatz kann aus Kunststoff bestehen, kann aber vorteilhafterweise aus einem Werkstoff gebildet werden, der biologisch abbaubar ist, wobei die Zeitspanne dieses biologischen Abbaus natürlich länger ist als zum Ausheilen eines Bruches des Oberarmes notwendig ist, so daß rechtzeitig der Nagel wieder entfernt werden kann. Durch den Einsatz des biologisch abbaubaren Materials wird aber erreicht, daß beim Aufbohren entstehende Materialsplitter nicht zu Entzündungen od. dgl. führen können.

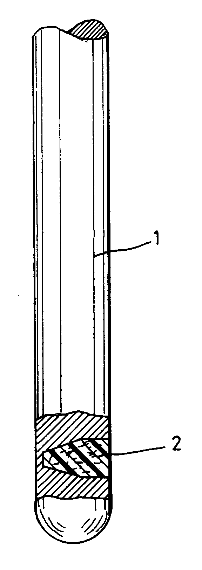

Ein Ausführungsbeispiel der Erfindung wird nachfolgend anhand der Zeichnung erläutert.

In der Zeichnung ist ein Teil eines Nagels 1 dargestellt, in dem im einen Endbereich ein Einsatz 2 vorgesehen ist, der bei dem dargestellten Ausführungsbeispiel in einer zylindrischen Bohrung angeordnet ist, die als Blindbohrung ausgebildet ist. Dieser Einsatz besteht aus einem Werkstoff, der biologisch abbaubar ist. Nunmehr kann nach Durchbohren der Cortikalis eine Schraube in diesen Einsatz eingeschraubt werden, die ein Festlegen des Nagels an der Cortikalis in diesem Endbereich des Nagels bewirkt.

## Patentansprüche

1. Aus Metall bestehender Marknagel (1) mit einem Einsatz (2) aus leicht aufbohrbarem oder schraubbaren Werkstoff, dadurch gekennzeichnet, daß der Einsatz (2) eine quer zur Längsachse des Marknagels (1) ausgerichtete Bohrung vollständig ausfüllt und wenigstens auf einer Seite des Nagels (1) nach außen mündet.

2. Marknagel nach Anspruch 1, dadurch gekennzeichnet, daß der Einsatz (2) aus biologisch abbaubarem Werkstoff besteht.

3. Marknagel nach Anspruch 1 oder 2, dadurch gekennzeichnet, daß der Einsatz (2) in einer oder mehreren Blindbohrungen im Nagel (1) angeordnet ist.

4. Marknagel nach Anspruch 1 oder 2, dadurch gekennzeichnet, daß der Einsatz (2) in einer zylindrischen Bohrung angeordnet ist, die sich in ihrem Endbereich konisch zur anderen Nagelseite verjüngt.

## Claims

1. A medullary nail (1) made of metal comprising an insert (2) of easily drillable or screw-threadable material, characterized in that the insert (2) completely fills a bore oriented perpendicularly to the longitudinal axis of the medullary nail (1) and opens outwards at least on one side of the nail (1).

2. A medullary nail according to claim 1, characterized in that the insert (2) consists of biologically degradable material.

3. A medullary nail according to claim 1 or claim 2, characterized in that the insert (2) is arranged in one or more blind bores in the nail (1).

4. A medullary nail according to claim 1 or claim 2, characterized in that the insert (2) is arranged in a cylindrical bore, the end area of which tapers conically towards the other nail side.

## Revendications

1. Clou médullaire (1) composé de métal avec un insert (2) en matériau facile à percer ou à visser, caractérisé en ce que l'insert (2) remplit entièrement un trou orienté perpendiculairement à l'axe longitudinal du clou médullaire (1) et débouche à l'extérieur sur au moins un côté du clou (1).

2. Clou médullaire selon la revendication 1, caractérisé en ce que l'insert (2) se compose de matériau biologiquement dégradable.

3. Clou médullaire selon la revendication 1 ou 2, caractérisé en ce que l'insert (2) est disposé dans un ou plusieurs trous borgnes dans le clou (1).

4. Clou médullaire selon la revendication 1 ou 2, caractérisé en ce que l'insert (2) est disposé dans un trou cylindrique qui se rétrécit en forme de cône dans son secteur d'extrémité en direction de l'autre côté du clou.
